# EUROPEAN PATENT APPLICATION

(11) **EP 4 574 960 A2**
(43) Date of publication of application: **25.06.2025**
(21) Application number: 24204817.1
(22) Date of filing: 04.10.2024
(51) Int. Cl.: C12M 1/06, C12M 1/34, C12M 1/36

(54) **FLEXIBLE FLOW AUTOMATIC STIRRING DEVICE AND ITS USE METHOD**

(30) Priority: 06.10.2023 CN 202311279505; 06.10.2023 CN 202311288855
(71) Applicant: Kunming Chao Tai Trade Co., Ltd., Kunming City, Yunnan Province 650225 (CN)
(72) Inventor: Wang, Wen Quan, Chengdu City, 610065 (CN); Tang, Lei, Chengdu City, 610065 (CN); Wang, Xiu, Chengdu City, 610065 (CN); Wei, Xin Yu, Chengdu City, 610065 (CN); Yan, Yan, Chengdu City, 610065 (CN); Zhang, Chao, Kunming City, 650225 (CN)
(74) Representative: Lang, Christian

(57) **Abstract**

The invention provides an automatic stirring device in the field of fluid machinery and bioreactors, focusing on enhancing fluid control efficiency and consistency in biological cultivation processes. It utilizes a flexible channel body or reactor body to facilitate the flow of nutrient solution along specific channels (such as S-shaped flexible channels or S-shaped loop channels), designed to reduce shear forces and achieve iso-level uniform flow. A power system and monitoring and control system are employed to regulate the flow of nutrient solution and monitor cultivation conditions in real-time, thus supporting various bioprocess cultivation requirements.

## Description

### FIELD OF THE INVENTION

The present invention relates to the technical field of fluid machinery and bioreactor apparatuses, particularly relates to a flexible flow automatic stirring device and its use method.

### BACKGROUND OF THE INVENTION

Traditional large-scale cell culture often uses paddle reactors, such as Stirred Tank Bioreactors (STR), where cells adhere to microcarrier sheets or are cultured in full suspension. The primary method involves the reactor's stirring paddles agitating the nutrient solution in the culture tank, causing the cells to adhere to the carrier or grow suspended within the bioreactor. However, this cultivation method results in low-quality cells, which do not meet the current needs for high-quality, high-efficiency cell culture, especially in response to sudden public health emergencies. The main issues are the direct stirring of adhered or suspended cells and their nutrient solution by the paddles, generating significant shear force that causes irreversible damage or direct death to the cells; the agitation of the nutrient solution creates a centrifugal effect, leading to non-uniform flow of the nutrient solution and uneven distribution of the nutrients needed for cell growth, resulting in poor cell quality. Some adherent cells used in production find it difficult to grow on traditional reactors and can only be cultured using traditional static methods, which have long culture cycles and uneven growth, failing to meet the demands for high-efficiency cultivation.

Currently, there are devices where cells are relatively fixed on the carrier, with the stirring paddles separated from the cell growth area, and the nutrient solution flows vertically from top to bottom or bottom to top through the cell reactor. This approach only addresses the problem of significant shear force generated during the rotation of the paddles, which damages or kills the cultured cells, and improves the tangential force on the cells caused by liquid flow. However, the flow rate of the nutrient solution across the culture dish's uniform cross-section is uneven and difficult to control, leading to uneven distribution of cells within the entire carrier cavity and varying amounts of nutrient absorption, ultimately resulting in inconsistent cell quality (a small number of cells expressing proteins well), failing to fundamentally solve this technical problem. In the face of public health emergencies, large-scale cultivation of high-quality cells in a short time is highly challenging, placing higher performance requirements on the consistency of cell quality in large-scale cultivation devices, which the existing devices have not fundamentally resolved. Moreover, existing cell culture tank devices lack dynamic monitoring methods for cells and nutrient solution states throughout the cell culture cycle, making it impossible to scientifically and accurately adjust the parameters of the cultivation device dynamically. During the cell culture process in existing stirring devices, excessive manual involvement in contacting individual culture dishes increases the likelihood of contamination in large-scale cultivation, reducing the reliability of cultivation quality. There is an urgent need for fully automatic stirring devices and cultivation processes with autonomous transport operations. Additionally, existing cell culture dish devices lack dynamic monitoring methods for cells and nutrient solution states throughout the cell culture cycle, making it impossible to scientifically and accurately adjust the parameters of the cultivation device dynamically from the source. These issues present significant challenges to the control processes for large-scale, high-quality cell culture.

The reasonable use of large adjustable flexible flow automatic stirring devices or flexible uniform flow culture dish automatic stirring devices can enable large-scale cultivation of high-quality cells, quantify dynamic monitoring and adjustment of cultivation device parameters, shorten the culture cycle, accelerate the development of reagents and drugs, and be used in the mass production of vaccines, reagents, and drugs. They can also fully automate operations such as sealing and transporting culture dishes, reducing manual involvement and truly achieving large-scale, high-quality, high-efficiency, and low-cost cell culture.

Therefore, how to achieve scale, high-efficiency, high-quality, and uniform cell culture is a technical problem that this field seeks to solve.

### SUMMARY OF THE INVENTION

One object of the present invention is to provide a flexible one-way circulating flow path in an S-shape formed by the reactor body. The energy conversion is achieved through the stirring and driving motion by the drive system, while the flipping mechanism achieves high-pressure sealing and automatic flipping of the cultivation device's cover. The real-time monitoring and adjustment system completes the monitoring and adjustment of cultivation parameters, enabling the achievement of various biological multi-process cultivation goals. This invention provides a large adjustable flexible flow automatic stirring device capable of uniform, high-efficiency, large-scale cell culture.

To achieve the above object, the technical solution adopted by the present invention is characterized by the large adjustable flexible flow automatic stirring device includes a reactor body, a drive system, a flipping mechanism, a monitoring and adjustment system, and a lifting mechanism. The reactor body includes a sealed cover, a culture tank, culture dishes, a turbulent plate, an arc surface plate, a partition plate, a flow channel tank, a bearing plate, and a conical chamber, realizing the function of nutrient solution flowing along the S-shaped flexible loop channel, featuring low shear force and iso-level uniform flow. The culture dish is provided with multiple regularly arranged honeycomb holes and mechanical grippers for convenient transport, with a ratio between inner diameter D0 and height H0 is 0.40 to 0.90. The drive system includes a stirring generator, a magnetic transmission shaft, a magnetic base, a magnetic connector, and a power source. The torque and rotational speed at the output end of the power source are transmitted sequentially to the magnetic connector, magnetic base, magnetic transmission shaft, and stirring generator. The stirring generator has an axial inflow and radial outflow mixed flow turbine structure, including multiple wing-shaped blades and flow channel outlets. The drive system achieves the function of stirring and driving the nutrient solution to flow in one direction from bottom to top along the S-shaped flexible loop channel. The flipping mechanism includes a guide rod, a slider, a connecting rod, and an actuator, achieving the function of high-pressure sealing and automatic flipping of the sealing cover. The monitoring and adjustment system includes multiple supply tubes, an oxygen sensor, a pH sensor, a liquid level detector, a rotational speed sensor, and a one-way solenoid valve, for real-time monitoring and/or adjusting the iso-level flow velocity, oxygen content, pH, and power source rotational speed of the nutrient solution in the culture tank. The lifting mechanism is provided with multiple sets of lifting legs, including an upper stud detachably connected to the bearing plate, a height adjustment stud, and a lower stud fixedly connected to the ground, bearing the device load and adjusting the position height and level state.

In a preferred embodiment of the present invention, the culture dish and the turbulent plate are detachably fixed, with the lower surface of the culture dish is flush with the lower surface of the turbulent plate, and the outer surface of the culture dish is sealed with the inner surface of the through hole, ensuring that the nutrient solution can only pass through the honeycomb holes from bottom to top. Multiple honeycomb holes arranged on the culture dish have center distances that are equidistant in both the circumferential and radial directions, and the multiple honeycomb holes are all located within the inner diameter D0 range of the culture dish. Multiple identical culture dishes are evenly arranged on the turbulent plate, with the inner circle of the centrally located culture dish coinciding with the horizontal projection circle of the arc ball surface, ensuring stable fluid flow in the central culture dish, and adjusting the circumferential arrangement radius R0 of the culture dish according to the cultivation process. Multiple supply tubes are fixed to the sealing cover through seamless welding, with the inlet of the supply tubes connected by a quick-release structure, achieving nutrient solution supply and cell ecological environment adjustment.

In a preferred embodiment of the present invention, the upper end surface of the turbulent plate is seamlessly connected to the lower end surface of the culture dish, the lower end surface is seamlessly connected to the upper end surface of the flow channel tank, and the circumference is connected to the inner wall of the culture tank. The turbulent plate includes multiple through-holes with equal diameter in the circumferential direction, reflux channels with the same structural dimensions, and bosses fixedly connected to the inner wall of the culture tank. The radius R2 of the reflux channels is more than four times the radius R1 of the culture dish.

In a preferred embodiment of the present invention, the arc surface plate is provided with an arc ball surface at the geometric center, multiple circumferentially positioned flexible flow channels, and an arc platform connected to the inner wall of the flow channel tank. The protruding surface of the arc ball surface is installed facing upwards to guide fluid flow, with the upward protrusion height of the arc ball surface determined according to the cultivation process. The diameter of the stirring generator flow path inlet is smaller than the center flow path hole of the partition plate, and the rotating axis of the stirring generator is coaxial with the geometric center axis of the stationary culture dishes, turbulent plate, and arc panel, with the flow path inlet plane at the lower end of the stirring generator coinciding with the lower plane of the partition plate.

In a preferred embodiment of the present invention, before use, the relative height adjustment between the upper stud and the lower stud is completed by rotating the adjustment stud of the lifting mechanism, ultimately achieving the level position adjustment of the entire cultivation device, followed by sterilization of the large adjustable flexible flow automatic stirring device. The actuator of the flipping mechanism is controlled for forward movement, uncovering the sealed cover of the cultivation device, and the culture dishes loaded with cell biological growth carriers in the form of floc sheets are fixedly placed into the through-holes of the turbulent plate by a mechanical arm. The actuator of the flipping mechanism is then controlled for reverse movement, installing the sealed cover on the upper end of the culture tank, completing the sealed environment of the cultivation device. The one-way solenoid liquid outlet valve is closed, the supply tube is opened to inject nutrient solution into the culture tank, and the power source is gradually started and the rotational speed adjusted, making the stirring generator gradually rotate, stir, and push the nutrient solution to flow towards the culture dishes along the S-shaped one-way flow path. The oxygen sensor, pH sensor, and liquid level detector probe of the analog quantity type are completely immersed in the nutrient solution, and by observing the value of the rotational speed sensor, adjusting the nutrient solution intake of the supply tube, and the rotational speed of the power source, a height difference is created between the nutrient solution in the culture dish and the nutrient solution in the outer ring cavity of the culture tank, with the nutrient solution in the culture dish flowing outwards. The monitoring and adjustment system performs real-time monitoring of the components, pH, and liquid level height of the nutrient solution, adjusting the cultivation parameters according to the cultivation process, achieving multi-process cultivation of different cell organisms.

Compared with the prior art, the present invention has the following beneficial effects. The present invention provides a novel large adjustable flexible flow automatic stirring device. (1) The reactor body forms an S-shaped one-way circulating flow path, where cell organisms in multiple culture dishes experience low shear force from nutrient solution flow, with minimal damage and large-scale production. (2) The use of an arc panel structure results in a minimal liquid level difference between the outer ring culture dishes and the central culture dishes, ensuring uniform nutrient solution flow speed on an equal height plane, leading to more uniform cell organism growth. (3) The device allows precise real-time measurement and control of rotational speed, pressure, and nutrient solution components, achieving digital cycle cultivation, high-efficiency cultivation, and high reliability. This setup is multifunctional, highly adaptable, and efficient, saving costs and ensuring performance and high-efficiency production for large-scale drug reagent development, optimization, and vaccine cultivation.

One object of the present invention is to provide a flexible uniform flow culture dish automatic stirring device and its usage method, which relies on a flexible flow channel body forming an S-shaped flexible loop channel. The power system drives energy conversion, and the biological culture parameters are monitored and adjusted in real time. This device ensures uniform and efficient cell biology cultivation.

To achieve the above object, the flexible uniform flow culture dish automatic stirring device includes a flexible flow channel body, a power system, a monitoring system, an adjustment system, a fixed base. The flexible flow channel body includes an outer tank cover plate, an outer tank, culture dishes, disk A, spherical plate, disk B, flow channel tube, and pad blocks, forming an S-shaped flexible loop channel that enables the one-way flow of nutrient liquid along the S-shaped flexible loop channel with low shear force and equal height plane equal-speed flow characteristics. The culture dishes are made of transparent material, evenly marked with multiple scale marks, with an inner diameter to height ratio of 0.55 to 0.95. The power system includes a power source, a magnetic coupling seat, a magnetic transmission shaft, and a turbine. The torque and speed of the power source are transmitted sequentially to the magnetic coupling seat, the magnetic transmission shaft, and the turbine. The power system adjusts the driving speed of the power source as needed for biological culture, making the turbine stir and push the nutrient liquid along the S-shaped flexible loop channel from bottom to top. The monitoring system includes a pressure sensor, a temperature sensor, a speed sensor, a test tube, and a display screen, which monitor the nutrient liquid pressure, temperature, equal height plane flow speed, and power source speed in real-time, providing data references for the adjustment system. The test tube has through slots, a polygonal head for position adjustment, and a plug to ensure a sealed environment. The adjustment system includes supply pipe A, supply pipe B, an inlet valve, and an outlet valve to adjust the nutrient liquid composition, temperature, and pressure in the culture dish. The fixed base includes a conical base for mounting the magnetic coupling seat, a rectangular base for bearing loads, and caster wheels with locking functions, supporting and moving the entire device load.

In a preferred embodiment of the present invention, the outer tank cover plate is provided with a threaded hole A for installing supply pipe A, a threaded hole B for installing supply pipe B, a fixing hole for fixing the test tube, and threads for connecting with the inner wall of the upper end of the outer tank. The positions of the test tube, supply pipe A, and supply pipe B can be adjusted in the axial direction of the outer tank cover plate to achieve high-pressure sealing. Threaded holes A and B are located outside the fixing hole for the test tube, allowing the nutrient liquid components supplied by supply pipe A and supply pipe B to be added to the annular flow channel formed between the outer tank and the culture dishes.

In a preferred embodiment of the present invention, the upper end of disk A is seamlessly connected with the lower end of the culture dish, the lower end is seamlessly connected with the upper end of the flow channel tube, and the circumference is connected to the inner wall of the outer tank. Disk A includes multiple honeycomb holes of equal diameter distributed in multiple concentric rings around the disk axis, and liquid return notches of the same structure and size located near the outer edge of the disk, with the center distances of the honeycomb holes on each ring being equidistant in both circumferential and radial directions, and the outermost ring diameter of the honeycomb holes being smaller than the inner diameter of the culture dish. The ratio of the radial size of the liquid return notches to the maximum radius of disk A (14) is 0.02-0.05.

In a preferred embodiment of the present invention, the spherical disk is provided with a central spherical surface at the geometric center, multiple flow channel openings at the outer edge, and multiple circumferential bosses connected to the inner wall of the flow channel cylinder, with the distance ratio between the spherical disk and disk A, and between the spherical disk and disk B being 0.65-0.95. The chord length of the central spherical surface is the same as the inner diameter of the culture dish, and the spherical radius is determined by the inner diameter of the culture dish.

In a preferred embodiment of the present invention, the turbine is a mixed flow structure with axial inflow and radial outflow, and has multiple wing-shaped blades, with the diameter of the flow channel inlet of the turbine being smaller than the flow channel hole at the center of disk B, with the rotating turbine and the stationary disk B coaxially aligned and closely fitted.

In a preferred embodiment of the present invention, a method for using the flexible uniform flow culture dish automatic stirring device includes the following steps. Step 1: Complete the sterilization of the flexible uniform flow culture dish automatic stirring device, close the one-way outlet valve, open the inlet valve to inject distilled water into the outer tank and the culture dish. Turn on and adjust the speed of the power source, the turbine rotates to stir the liquid in the culture dish into the annular cavity between the outer tank and the culture dish, and check if the monitoring system is working properly.

Step 2: After completing the preparation work, aseptically remove the outer tank cover plate and evenly place fibrous carrier flocculent sheets in the culture dish. Then, seal and install the outer tank cover plate on the upper end of the outer tank again; reinject the nutrient solution into the culture dish through the inlet valve, turn on and adjust the speed of the power source, the turbine rotates to stir and push the nutrient solution in the culture dish into the annular cavity between the outer tank and the culture dish.

Step 3: Rotate and adjust the test tube so that the probe of the pressure sensor installed at the lower end of the test tube is fully immersed in the nutrient solution, adjust the opening state of the outlet valve and the speed of the turbine, to create a height difference between the nutrient solution inside the culture dish and the nutrient solution in the annular cavity outside the culture dish.

Step 4: Adjust the test tube so that the probe of the pressure sensor is positioned at multiple horizontal levels in the culture dish, record the pressure data at different levels; when the monitored pressure value of the pressure sensor exceeds the pressure tolerance level of the cultured cells, adjust the speed of the turbine and the opening state of the outlet valve according to the alarm displayed on the display screen.

Step 5: After a certain period of cell culture, use an external peristaltic pump to connect supply pipe A and supply pipe B to quantitatively replenish the nutrient solution; throughout the entire cell culture cycle, control the opening state of the outlet valve, the speed of the turbine, and the nutrient replenishment interval time to achieve multi-process cultivation of different cell types.

Compared with the prior art, the present invention has the following beneficial effects. The present invention provides a novel flexible uniform flow culture dish automatic stirring device and usage method. The flexible flow channel body forms an S-shaped one-way circulating flow channel. The cells in the culture dish area experience low shear force from the nutrient liquid flow, resulting in minimal damage and large-scale production. (2) Using a spherical plate structure, the nutrient liquid flow speed in the equal height plane of the culture dishes is uniform, leading to more homogeneous cell growth. (3) The device can accurately measure, display, and control speed, pressure, temperature, and nutrient liquid composition in real time, achieving digital cyclic cultivation with high efficiency and reliability. This setup offers multiple functions, strong applicability, and high efficiency, saving costs, ensuring performance, and promoting production in pharmaceutical development, reagent optimization, and vaccine cultivation.

### BRIEF DESCRIPTION OF THE DRAWINGS

FIG.1 is a schematic structural diagram of the large adjustable flexible flow automatic stirring device of the present invention.
FIG.2 is a partial enlarged view of FIG.1.
FIG.3 is a Cross-sectional view of the large adjustable flexible flow automatic stirring device of the present invention.
FIG.4 is a A-A view of FIG.3.
FIG.5 is a B-B view of FIG.3.
FIG.6 is a schematic structural diagram of the flexible uniform flow culture dish automatic stirring device and its usage method of the present invention.
FIG.7 is a front view of the present invention.
FIG.8 is a A-A view of FIG.7.
FIG.9 is a top view of FIG.7.
FIG.10 is a schematic structural diagram of disk A of the present invention;
FIG.11 is a schematic structural diagram of the spherical plate of the present invention.
FIG.12 is a B-B view of FIG.11.
FIG.13 is a schematic structural diagram of the test tube assembly of the present invention.
FIG.14 is a schematic structural diagram of the outer tank cover plate of the present invention.
FIG.15 is a schematic structural diagram of the turbine of the present invention.
Wherein: 1'-reactor body; 2'-drive system; S'-flipping mechanism; 4'-monitoring and adjustment system; 5'-lifting mechanism; 11'-sealing cover; 12'-culture tank; 13'-culture dish; 14'-turbulent plate; 15'-arc surface plate; 16'-partition plate; 17'-flow channel tank; 18'-bearing plate; 19'-conical chamber; 21'-stirring generator; 22'-magnetic transmission shaft; 23'-magnetic base; 24'-magnetic connector; 25'-power source; 31'-guide rod; 32'-slider; 33'-connecting rod; 34'-actuator; 41'-supply pipe; 42'-oxygen sensor; 43'-pH sensor; 44'-liquid level detector; 45'-rotational speed sensor; 46'-one-way solenoid valve; 51'-upper screw stud; 52'-adjustment screw stud; 53'-lower screw stud; 111'-guide stud; 112'-hinge seat; 113'-fixed seat; 121'-slider seat; 131'-honeycomb hole; 132'-mechanical gripper; 141'-through hole; 142'-reflux channel; 143'-boss; 151'-arc ball surface; 152'-flexible flow channel; 153'-arc platform; 211'-wing-shaped blade; 212'-flow channel outlet; 321'-upper arm; 322'-connecting rod end; 323'-lower arm; 331'-U-shaped seat; 332'-fixed end; 341'-telescoping rod; 342'-telescoping cylinder; 1-flexible flow channel body; 2-power system; 3-monitoring system; 4-adjustment system; 5-fixed base; 11-outer tank cover plate; 12-outer tank; 13-culture dish; 14-disk A; 15-spherical plate; 16- disk B; 17-flow channel cylinder; 18-spacer; 21-power source; 22-magnetic coupling seat; 23-magnetic transmission shaft; 24-turbine; 31-pressure sensor; 32-temperature sensor; 33-rotational speed sensor; 34-test tube; 35-display screen; 41-supply pipe A; 42-supply pipe B; 43-inlet valve; 44-outlet valve; 51-conical base; 52-rectangular base; 53-caster wheel; 111-threaded hole A; 112-threaded hole B; 113-fixing hole; 114-thread; 141-honeycomb hole; 142-reflux notch; 151-central spherical surface; 152-flow channel opening; 153-circumferential boss; 241-wing-shaped blade; 242-flow channel inlet; 243-flow channel outlet; 341-waist-shaped hole; 342-polygonal square head; 343-plug; and 521-fixing hole.

### DETAILED DESCRIPTION OF THE PREFERRED EMBODIMENT

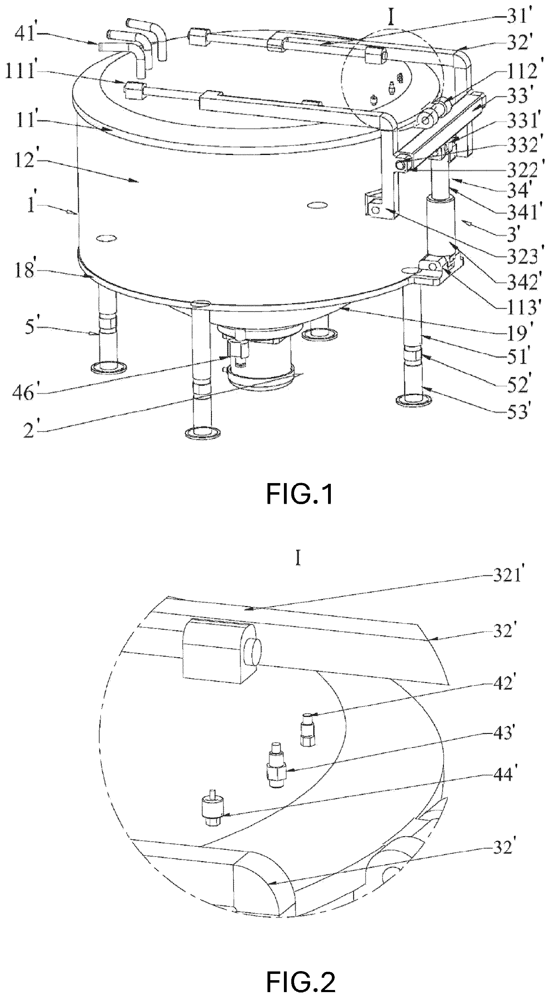

In one embodiment, as shown in FIG.1 to FIG.5, the large adjustable flexible flow automatic stirring device includes a reactor body 1', a drive system 2', a flipping mechanism 3', a monitoring and adjustment system 4', and a lifting mechanism 5'. The reactor body 1' includes a sealing cover 11', a culture tank 12', a culture dish 13', a turbulent plate 14', a curved surface panel 15', a partition plate 16', a flow channel tank 17', a bearing plate 18', and a conical chamber 19'. This arrangement achieves the function of nutrient flow along an S-shaped flexible loop channel, featuring low shear force and iso-level uniform flow. The culture dish 13' is provided with multiple regularly arranged honeycomb holes 131' and mechanical grippers 132' for convenient transport, with the ratio between its inner diameter D0 and the height H0 is 0.40-0.90. The drive system 2' includes a stirring generator 21', a magnetic transmission shaft 22', a magnetic base 23', a magnetic connector 24', and a power source 25'. The torque and rotational speed at the output end of the power source 25' are sequentially transmitted to the magnetic connector 24', magnetic base 23', magnetic transmission shaft 22', and stirring generator 21'. The stirring generator 21' is a mixed flow turbine structure with axial inflow and radial outflow, including multiple wing-shaped blades 211' and flow channel outlets 212'. The drive system 2' realizes the function of stirring and driving the nutrient solution to flow in one direction from bottom to top along the S-shaped flexible loop channel. The flipping mechanism 3' includes a guide rod 31', a slider 32', a connecting rod 33', and an actuator 34', realizing the functions of high-pressure sealing and automatic flipping of the sealing cover 11'. The monitoring and adjustment system 4' includes multiple supply pipes 41', an oxygen sensor 42', a pH sensor 43', a liquid level detector 44', a rotational speed sensor 45', and a one-way solenoid valve 46', for real-time monitoring and/or adjusting the iso-level flow velocity, oxygen content, pH, and the power source 25' rotational speed in the culture tank 12'. The lifting mechanism 5' is provided with multiple sets of lifting legs, including an upper screw stud 51' detachable connected to the bearing plate 18', a height adjustment screw stud 52', and a lower screw stud 53' fixedly connected to the ground, bearing the device load and adjusting the position height and level state.

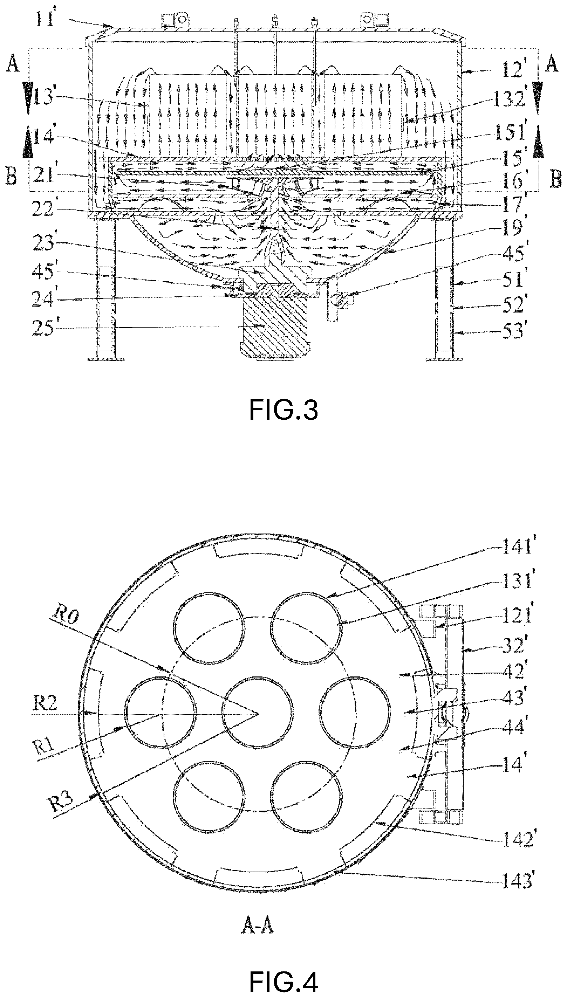

As shown in FIG.1 and FIG.4, in one embodiment, the flipping mechanism 3' is a connecting rod-slider mechanism, wherein the actuator 34' is a telescoping hydraulic cylinder with fixed hinges at both ends. The upper arm 321' of the slider 32' is slidingly connected to the circular guide rod 31', while the lower arm 323' of the slider 32' is hinged to the slider seat 121'. The hinge seat 112' of the sealing cover 11' is connected to the culture tank 12' via a pin shaft. The two connecting rod ends 322' are fixedly connected to the fixed ends 332' of the rectangular connecting rod 33', transmitting torque. The U-shaped seat 331' in the middle of the rectangular connecting rod 33' is hinged to the telescoping rod 341' of the actuator 34', and the base of the telescoping cylinder 342' of the actuator 34' is hinged to the fixed seat 113'. When sealing is needed between the sealing cover 11' and the culture tank 12', the telescoping rod 341' of the actuator 34' extends, and the actuator 34' rotates counterclockwise. When the culture tank 12' needs to be opened, the telescoping rod 341' of the actuator 34' retracts, achieving fully automatic flipping of the sealing cover 11'.

As shown in FIG.3 and FIG.4, in one embodiment, to reduce contamination during the cultivation process and improve cultivation quality, and to enable automatic transfer of the cultivation medium by a robotic arm, the culture dish 13' is detachably fixed to the turbulent plate 14' . The lower surface of the culture dish 13' is flush with the lower surface of the turbulent plate 14', and a high-pressure seal is maintained between the outer surface of the culture dish 13' and the inner surface of the through hole 141' of the turbulent plate 14'. The multiple honeycomb holes 131' on the culture dish 13' are equally spaced in both circumferential and radial directions, and all the honeycomb holes 131' are located within the inner diameter D0 range of the culture dish 13'. The flowing nutrient solution can only flow from the bottom to the top through the honeycomb holes 131' into the culture dish 13', and then overflow outward through the upper opening of the culture dish 13' into the culture tank 12'. In one embodiment, multiple culture dishes 13' with the same structure are evenly arranged on the turbulent plate 14'. The central culture dish 13' has a circular projection that coincides with the arc ball surface 151' of the arc surface plate 15' on a horizontal plane. The circumferential arrangement radius R0 of the culture dish 13' is adjusted according to the cultivation process.

As shown in FIG.1 to FIG.3, in one embodiment, for real-time monitoring and adjustment of cultivation parameters, the supply pipe 41' in the monitoring and adjustment system 4' is connected to the sealing cover 11'. Three supply pipes 41' are for feeding, oxygen supply, and exhaust. The nutrient solution fed through the supply pipe flows into the annular reflux channel between the culture tank 12' and the culture dish 13', ensuring thorough mixing of the nutrient solution components during the reflux process. The oxygen content of the cell growth environment is precisely controlled to 95% by separately controlling the oxygen supply pipe and the feeding pipe. The oxygen sensor 42' is an optical dissolved oxygen sensor InPro6860i/12/120/mA/HD, featuring a hydrophilic surface modified to prevent bubble accumulation, providing a highly stable measurement signal, with digital (MODBUS, RS-485) and analog (mA) signal outputs. The pH sensor 43' is a pH electrode type InPro3100, used for measuring the pH level of the cultivation medium during the cultivation process. The liquid level detector 44' is a resistance-type analog signal output sensor, with a characteristic that the output signal increases with the rising liquid level.

As shown in FIG.3, in one embodiment, the upper end surface of the turbulent plate 14' is seamlessly connected to the lower end surface of the culture dish 13', the lower end surface is seamlessly connected to the upper end surface of the flow channel tank 17', and the circumferential edge is connected to the inner wall of the culture tank 12'. An annular chamber with fixed dimensions is formed between the culture dish 13' and the turbulent plate 14'. The turbulent plate 14' includes eight uniformly arranged through holes 141' of equal diameter, reflux channels 142' of the same structural size, and a boss 143' fixedly connected to the inner wall of the culture tank 12'. To achieve one-way flow of the nutrient solution along the S-shaped flexible channel and ensure performance, the radius R2 of the reflux channel 142' is more than four times the radius R1 of the culture dish 13'.

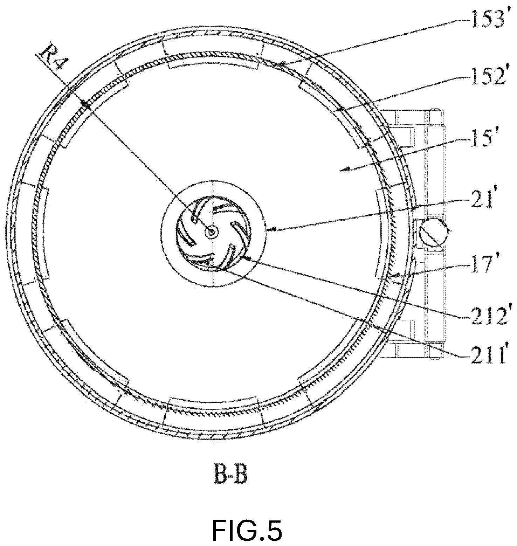

As shown in FIG. 3 and FIG.5, in one embodiment, the arc surface plate 15' has an arc ball surface 151' at its geometric center, multiple flexible flow channels 152' at circumferential positions, and an arc platform 153' connected to the inner wall of the flow channel tank 17'. The arc ball surface 151' of the arc surface plate 15' is mounted protruding upwards to guide fluid flow, with the upward protruding height of the arc ball surface 151' determined according to the cultivation process. As shown in FIG.5, the stirring generator 21' is a mixed-flow turbine structure with axial inflow and radial outflow, having six wing-shaped blades 211'. The diameter of the flow channel outlet 212' of the stirring generator 21' is smaller than the diameter of the flow channel hole at the center of the partition plate 16'. The rotating stirring generator 21' and the stationary flow channel hole of the partition plate 16' are coaxial with a gap fit, ensuring thorough and uniform stirring of the supplemental nutrient solution and propulsion of the nutrient solution flow.

In one embodiment, the large adjustable flexible flow automatic stirring device utilizes the reactor body 1' components and the coupling drive system 2' to achieve the function of low shear force for one-way flow of nutrient solution along the S-shaped flexible channel. The arc ball surface 151' in the reactor body 1' alters the flow rate within the S-shaped flexible channel, ensuring iso-level uniform flow in multiple culture dishes 13'. This not only ensures high-quality cell cultivation but also allows for adjustments in the relative positioning and number of culture dishes 13', thereby enhancing both the quantity and quality of the cultured cells. The flipping mechanism 3' reduces manual operation, with the sealing cover 11' achieving automatic closure to improve reliability and ensure cultivation quality. The monitoring and adjustment system 4' provides visualization, detailed monitoring, management, and control of the cultivation process and multi-process cultivation goals, preventing operator errors and improving cultivation efficiency. The lifting mechanism 5' adjusts the entire position height and level state of the device, with reliable structure and strong practicality and operability.

In the aforementioned embodiment, the object of the present invention for the large adjustable flexible flow automatic stirring device can be achieved, and technicians in the field can make selections based on actual conditions.

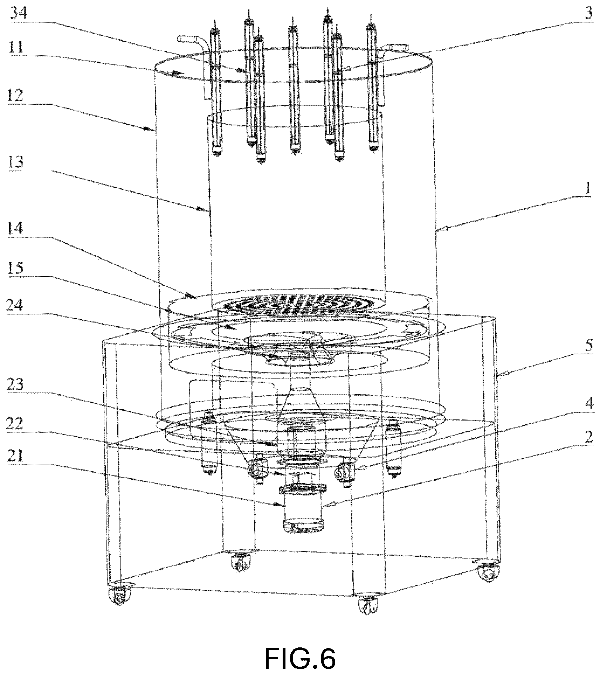

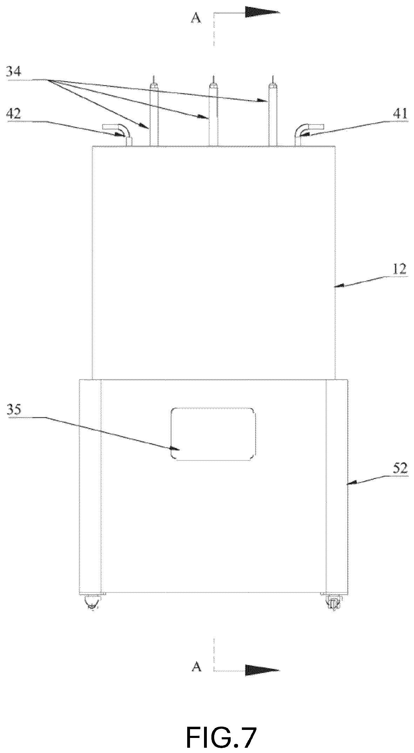

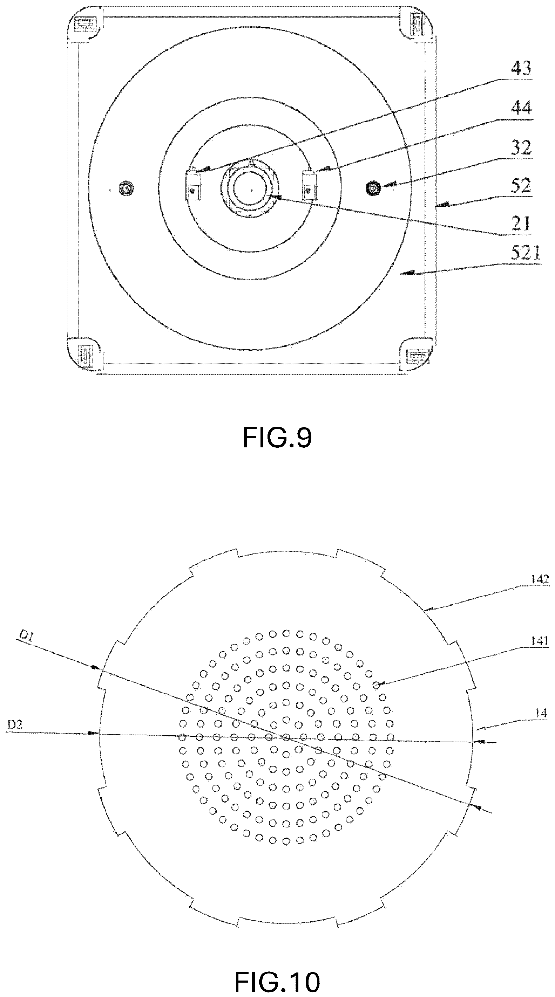

As shown in FIG.6 to FIG.9, the flexible uniform flow culture dishes automatic stirring device includes a flexible flow channel body 1, a power system 2, a monitoring system 3, an adjustment system 4, and a fixed base 5. The flexible flow channel body 1 includes an outer tank cover plate 11, an outer tank 12, culture dishes 13, disk A 14, spherical disk 15, disk body B 16, flow channel cylinder 17, and spacer 18, together forming an S-shaped flexible loop channel that realizes the one-way flow of nutrient solution with low shear force and uniform flow. The culture dish 13 is made of a transparent material with multiple scale marks evenly distributed on the outer wall, and the ratio of inner diameter to height size is 0.55-0.95, such as 0.6. The power system 2 includes a power source 21, a magnetic coupling seat 22, a magnetic transmission shaft 23, and a turbine 24. In one embodiment, the power source 21 is a stepless speed regulation motor, which transmits the motor's output torque and speed sequentially to the magnetic coupling seat 22, magnetic transmission shaft 23, and turbine 24 through a spline connection. The driving speed is adjusted according to the cultivation needs, causing the turbine 24 to stir and push the nutrient solution to flow upward along the S-shaped flexible loop channel. The monitoring system 3 includes a pressure sensor 31, a temperature sensor 32, a rotational speed sensor 33, a test tube 34, and a display screen 35, which monitor in real-time the pressure, temperature, uniform flow rate of the nutrient solution in the culture dishes, and the rotational speed of the power source. The test tube 34 is connected to the outer tank cover plate 11 via an external thread. The test tube 34 is provided with a through waist-shaped hole 341, a polygonal square head 342 for position adjustment, and a plug 343 for sealing. The adjustment system 4 includes supply pipe A 41, supply pipe B 42, an inlet valve 43, and an outlet valve 44. The fixed base 5 includes a conical base 51 for mounting the magnetic coupling seat 22, a rectangular base 52 for bearing load, and caster wheels 53 with locking function. The conical base 51 is fixed on the rectangular base 52 through fixing holes 521, bearing and moving the entire device load.

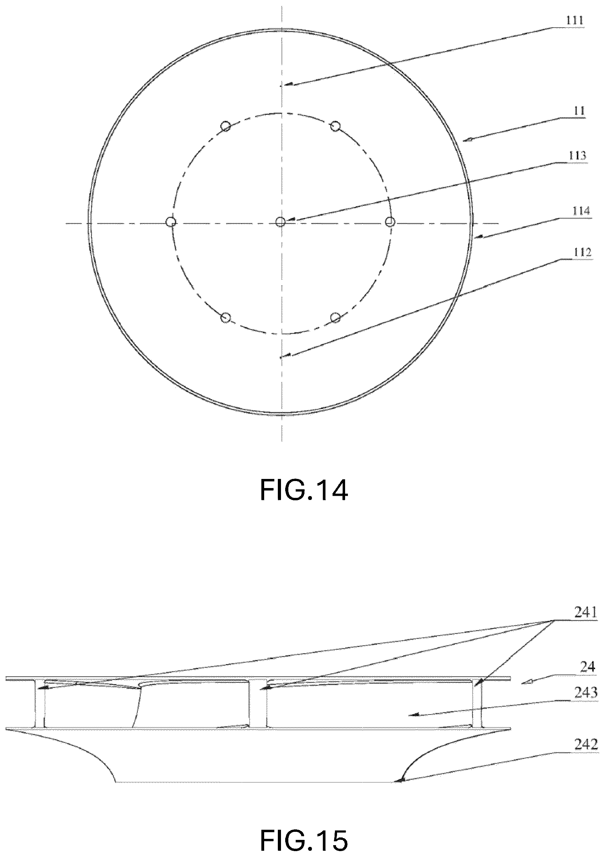

As shown in FIG.6 and FIG.14, in one embodiment, the outer tank cover plate 11 is provided with a threaded hole A 111 for installing supply pipe A 41, a threaded hole B 112 for installing supply pipe B 42, a fixing hole 113 for fixing the test tube 34, and a thread 114 for connecting with the inner wall of the upper end of the outer tank 12. The threaded holes A 111 and threaded holes B 112 are located on the outer ring of the fixing hole 113. The positions of the test tube 34, supply pipe A 41, and supply pipe B 42 can all be adjusted axially on the outer tank cover plate 11 and achieve high-pressure sealing, and they are positioned outside the projected horizontal plane of the culture dish 13. After installing supply pipe A 41 and supply pipe B 42 on the outer tank cover plate 11, the nutrient solution supplied by the supply pipes flows into the annular reflux flow channel between the outer tank 12 and the culture dish 13, allowing the nutrient solution components to be fully and evenly mixed during the reflux process.

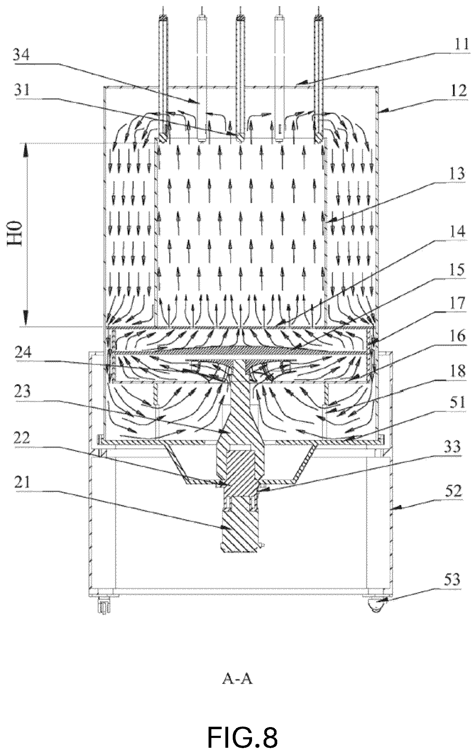

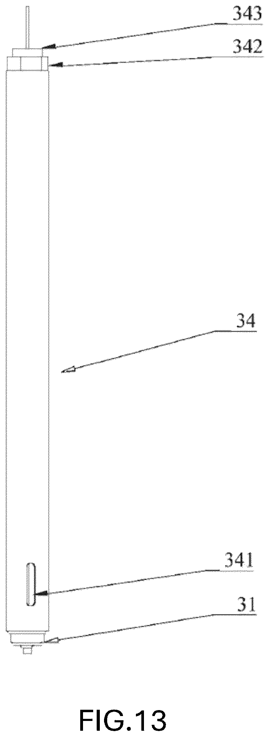

As shown in FIG.8, FIG.10 and FIG.13, in one embodiment, the upper end surface of the disk A 14 is seamlessly connected to the lower end surface of the culture dish 13, the lower end surface is seamlessly connected to the upper end surface of the flow channel cylinder 17, and the circumferential side is connected to the inner wall of the outer tank 12. Disk A 14 includes multiple honeycomb holes 141 of equal diameter distributed in multiple concentric rings around the disk axis, and liquid return notches 142 of the same structure and size evenly distributed near the outer edge of the disk. The center distance of the honeycomb holes 141 on each ring is equidistant in both circumferential and radial directions, and the outermost ring diameter of honeycomb holes 141 is smaller than the inner diameter of the culture dish 13. The ratio of the radial size of the liquid return notches 142 to the maximum radius of disk A 14 is 0.02-0.05. In the embodiment, the ratio of the difference between the radial dimensions D1 and D2 of the return notches 142 to the maximum radius of the disk A 14 is such as 0.03.

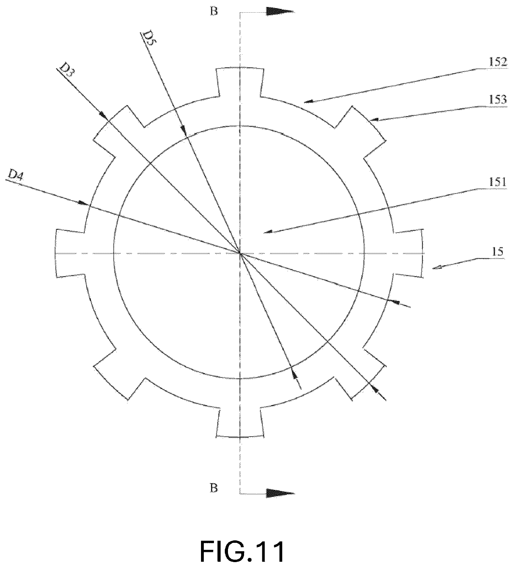

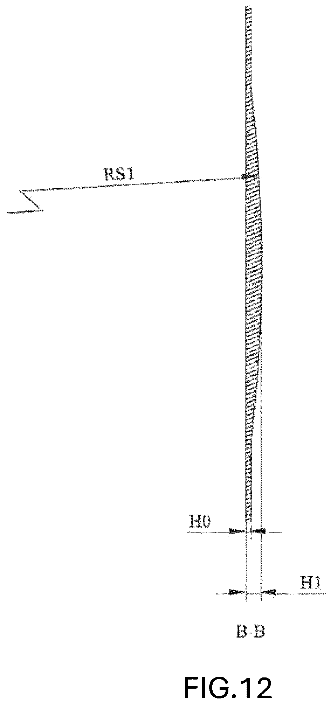

As shown in FIG.8, FIG.11, and FIG.12, in one embodiment, the spherical disk 15 is provided with a central spherical surface 151 at the geometric center and eight flow channel openings 152 at the outer edge. The eight circumferential bosses 153 of the spherical disk 15 are fixedly connected to the inner wall of the flow channel cylinder 17, bearing the shear force generated by the rotation of the lower turbine 24 on the fluid. The distance ratio between the spherical disk 15 and disk A 14, and between the spherical disk 15 and disk B 16 being 0.65-0.95. In the embodiment, the maximum planar distance between the spherical disk 15 and disk A 14 and disk B 16 is 0.68. The chord length of the central spherical surface 151 is the same as the inner diameter of the culture dish 13, and the inner diameter of the culture dish 13 determines the difference between the spherical radius RS1, H1, and H0.

As shown in FIG.15, the turbine 24 is a mixed flow structure with axial inflow and radial outflow and has multiple wing-shaped blades 241. The diameter of the flow channel inlet 242 of the turbine 24 is smaller than the flow channel hole at the center position of disk B 16. The rotating turbine 24 and the stationary disk B 16 are coaxially aligned and closely fitted. The liquid drawn in through the flow channel inlet 242 flows out through six flow channel outlets 243, achieving sufficient and even stirring and energy conversion of the nutrient solution.

As shown in FIG.6, FIG.8, and FIG.13, in one embodiment, the method for using the automatic stirring device for flexible iso-velocity flow culture dishes includes the following steps. First step: Move the device to the working area, complete the sterilization of the flexible uniform flow culture dish automatic stirring device, close the one-way outlet valve 44, open the inlet valve 43 to inject distilled water into the outer tank 12 and culture dishes 13. Turn on the power source 21 at low speed for warming up; gradually adjust the speed, and the turbine 24 rotates to stir the liquid in the culture dishes 13 into the annular cavity between the outer tank 12 and the culture dish 13, checking whether the entire device is functioning normally. If the cultivation device is working normally, proceed to the next step; otherwise, shut down and inspect the entire device.

Second step: After completing the preparation work, rotate the outer tank cover plate 11 aseptically to remove it from the outer tank 12, and evenly place fibrous carrier flocculent sheets in the culture dishes 13. Then, seal and install the outer tank cover plate 11 on the upper end of the outer tank 12 again; reinject the nutrient solution into the culture dishes 13 through the inlet valve 43, turn on and adjust the speed of the power source 21 at low speed, and the turbine 24 rotates to stir and push the nutrient solution in the culture dish 13 into the annular cavity between the outer tank 12 and the culture dish 13.

Third step: Adjust the height of the test tube 34 by rotating the polygonal square head 342, so that the probe of the pressure sensor 31 installed at the lower end of the test tube 34 is fully immersed in the nutrient solution. Adjust the opening state of the outlet valve 44 and the speed of the turbine 24 so that the nutrient solution inside the culture dishes 13 and the nutrient solution in the annular cavity outside the culture dishes 13 reach the height difference required by the cultivation process.

Fourth step: Adjust the height of the test tube 34 so that the probe of the pressure sensor 31 installed on the test tube 34 are located at three different levels in the culture dishes 13, recording the pressure data at different levels. When the monitored pressure value of the pressure sensor 31 exceeds the pressure tolerance level of the cell culture process, an alarm is displayed on the display screen 35, and the speed of the turbine 24 and the opening state of the outlet valve 44 are comprehensively adjusted to improve the cultivation process.

Fifth step: After culturing the cells for a certain period, use an external peristaltic pump to supply pipe A 41 and supply pipe B 42 to quantitatively replenish the nutrient solution. Throughout the entire cell culture cycle, control the opening state of the outlet valve 44, the speed of the turbine 24, and the nutrient replenishment interval time to achieve multi-process cultivation of different cell types.

In one embodiment, the flexible constant velocity flow culture dish automatic stirring device and its method of use, through the combination of the flexible flow channel body component 1, the continuously variable speed motor power source, the magnetic coupler, and the turbine using a keyed connection and magnetic coupling transmission mode series-installed power system 2, achieve the function of one-way small shear force flow of the nutrient solution along the S-shaped flexible loop channel. At the same time, the spherical plate in the flexible flow channel body 1 changes the flow velocity in the S-shaped flow channel, ensuring uniform flow at the high surface in the culture dish 13, thus achieving high-quality cell culture. The monitoring system 3 and the adjustment system 4 realize visualized, refined monitoring, management, and control of the culture process, avoiding operator errors and improving culture efficiency.

In the aforementioned embodiment, the object of the present invention for the flexible uniform flow culture dish automatic stirring device and its method of use can be achieved, and technicians in the field can make selections based on actual conditions.

The large adjustable flexible flow automatic stirring device and the flexible constant velocity flow culture dish automatic stirring device and its method of use provided by this invention can be applied to pharmaceutical product development and mass production. In one embodiment, the large adjustable flexible flow automatic stirring device and the flexible constant velocity flow culture dish automatic stirring device and its method of use belong to bioreactor technology and fluid machinery technology. This invention provides a new large-scale cell biology culture method. By designing the structure of the culture device, selecting the S-shaped flexible loop flow channel, the power transmission route, and the implementation method of the real-time monitoring and adjustment system, it has the functions of multi-process, large-scale culture, easy operation, reliable structure, and high culture quality. This invention provides a new method for cell biology culture. By designing the structure of the flexible constant velocity flow culture dish automatic stirring device, adopting the S-shaped flexible loop flow channel, axially series-connected power system to transmit torque and speed, and real-time monitoring and adjustment of culture parameters, it has the functions of multi-process culture, easy operation, reliable structure, and high culture quality.

The above content is only an example and description of the structure of the invention. Technical personnel in the field can make various modifications or supplements to the specific embodiments described or adopt similar methods as long as they do not deviate from the structure of the invention or exceed the scope defined by the claims, which should all be within the protection scope of the present invention.

## Claims

1. A large adjustable flexible flow automatic stirring device, comprising a reactor body (1'), a drive system (2'), a flipping mechanism (3'), a monitoring and adjustment system (4'), and a lifting mechanism (5'), **characterized by**:
the reactor body (1') includes a sealing cover (11'), a culture tank (12'), a culture dish (13'), a turbulent plate (14'), an arc surface plate (15'), a partition plate (16'), a flow channel tank (17'), a bearing plate (18'), and a conical chamber (19'), realizing the function of nutrient solution flowing along an S-shaped flexible loop channel with features of low shear force and iso-level uniform flow; the culture dish (13') is provided with multiple regularly arranged honeycomb holes (131') and mechanical grippers (132') for convenient transport, and the ratio between its inner diameter D0 and height H0 is 0.40-0.90; the drive system (2') includes a stirring generator (21'), a magnetic transmission shaft (22'), a magnetic base (23'), a magnetic connector (24'), and a power source (25'), the torque and rotational speed at the output end of the power source (25') are transmitted sequentially to the magnetic connector (24'), magnetic base (23'), magnetic transmission shaft (22'), and stirring generator (21'); the stirring generator (21') is a mixed flow turbine structure with axial inflow and radial outflow, comprising multiple wing-shaped blades (211') and flow channel outlets (212'); the drive system (2') realizes the function of stirring and driving the nutrient solution to flow in one direction from bottom to top along the S-shaped flexible loop channel; the flipping mechanism (3') includes a guide rod (31'), a slider (32'), a connecting rod (33'), and an actuator (34'), realizing the functions of high-pressure sealing and automatic flipping of the sealing cover (11'); the monitoring and adjustment system (4') includes multiple supply pipes (41'), an oxygen sensor (42'), a pH sensor (43'), a liquid level detector (44'), a rotational speed sensor (45'), and a one-way solenoid valve (46'), for real-time monitoring and/or adjusting the iso-level flow velocity, oxygen content, pH, and power source (25') rotational speed in the culture tank (12'); the lifting mechanism (5') is provided with multiple sets of lifting legs, including an upper stud (51') detachably connected to the bearing plate (18'), a height adjustment stud (52'), and a lower stud (53') fixedly connected to the ground, bearing the device load and adjusting the position height and level state.

2. The large adjustable flexible flow automatic stirring device according to claim 1, **characterized in that**: the culture dish (13') and the turbulent plate (14') are detachably fixed, the lower surface of the culture dish (13') is flush with the lower surface of the turbulent plate (14'), and the outer surface of the culture dish (13') is sealed with the inner surface of the through hole (141'); multiple honeycomb holes (131') arranged on the culture dish (13') have center distances that are equidistant in both the circumferential and radial directions, and the multiple honeycomb holes (131') are all located within the inner diameter D0 range of the culture dish (13'), allowing the nutrient solution to pass through the honeycomb holes (131') only from bottom to top; multiple identical culture dishes (13') are evenly arranged on the turbulent plate (14'), with the inner circle of the centrally located culture dish (13') coinciding with the horizontal projection circle of the arc ball surface (151') protruding from the arc surface plate (15'), and the circumferential arrangement radius R0 of the culture dish (13') is adjusted according to the cultivation process.

3. The large adjustable flexible flow automatic stirring device according to claim 1, **characterized in that**: the upper end surface of the turbulent plate (14') is seamlessly connected to the lower end surface of the culture dish (13'), the lower end surface is seamlessly connected to the upper end surface of the flow channel tank (17'), and the circumference is connected to the inner wall of the culture tank (12'); the turbulent plate (14') includes multiple uniformly distributed through holes (141') with equal diameters, reflux channels (142') with the same structural dimensions, and bosses (143') fixedly connected to the inner wall of the culture tank (12'); the radius R2 of the reflux channels (142') is more than four times the radius R1 of the culture dish (13').

4. The large adjustable flexible flow automatic stirring device according to claim 1, **characterized in that**: the arc surface plate (15') is provided with an arc ball surface (151') at the geometric center, multiple circumferentially positioned flexible flow channels (152'), and an arc platform (153') connected to the inner wall of the flow channel tank (11'); the protruding surface of the arc ball surface (151') is installed facing upward to guide fluid flow, and the upward protruding height of the arc ball surface (151') is determined according to the cultivation process.

5. A flexible uniform flow culture dish automatic stirring device, comprising a flexible flow channel body (1), a power system (2), a monitoring system (3), an adjustment system (4), and a fixed base (5), **characterized by**:
the flexible flow channel body (1) includes an outer tank cover plate (11), an outer tank (12), a culture dish (13), disk A (14), a spherical disk (15), disk B (16), a flow channel cylinder (17), and a spacer (18), forming an S-shaped flexible loop channel, realizing the function of the nutrient solution flowing in one direction along the S-shaped flexible loop channel with low shear force and uniform flow; the culture dish (13) is made of transparent material with evenly arranged scale markings on its outer wall, and the ratio of its inner diameter to height is 0.55-0.95; the power system (2) includes a power source (21), a magnetic coupling seat (22), a magnetic transmission shaft (23), and a turbine (24), the torque and rotational speed of the power source (21) are sequentially transmitted to the magnetic coupling seat (22), magnetic transmission shaft (23), and turbine (24); the drive speed is adjusted according to the cultivation needs, enabling the turbine (24) to stir and push the nutrient solution to flow from bottom to top along the S-shaped flexible loop channel; the monitoring system (3) includes a pressure sensor (31), a temperature sensor (32), a rotational speed sensor (33), a test tube (34), and a display screen (35), for real-time monitoring of the pressure, temperature, uniform flow speed of the nutrient solution in the culture dish, and the rotational speed of the power source; the test tube (34) is provided with a through waist-shaped hole (341), a polygonal square head (342) for position adjustment, and a plug (343) for sealing, and the test tube (34) is connected to the outer tank cover plate (11) via an external thread; the adjustment system (4) includes supply pipe A (41), supply pipe B (42), an inlet valve (43), and an outlet valve (44); the fixed base (5) includes a conical base (51) for mounting the magnetic coupling seat (22), a rectangular base (52) for bearing loads, and caster wheels (53) with locking function, for bearing and moving the entire device load.

6. The flexible uniform flow culture dish automatic stirring device according to claim 5, **characterized in that**: the outer tank cover plate (11) is provided with threaded hole A (111) for installing supply pipe A (41), threaded hole B (112) for installing supply pipe B (42), a fixing hole (113) for fixing the test tube (34), and threads (114) for connecting with the inner wall of the upper end of the outer tank (12), with threaded hole A (111) and threaded hole B (112) located on the outer ring of the fixing hole (113); the positions of the test tube (34), supply pipe A (41), and supply pipe B (42) can all be adjusted axially along the outer tank cover plate (11) and achieve high-pressure sealing, and they are positioned outside the projected horizontal plane of the culture dish (13).

7. The flexible uniform flow culture dish automatic stirring device according to claim 5, **characterized in that**: the upper end surface of disk A (14) is seamlessly connected to the lower end surface of the culture dish (13), the lower end surface is seamlessly connected to the upper end surface of the flow channel cylinder (17), and the circumference is connected to the inner wall of the outer tank (12); disk A (14) includes multiple honeycomb holes (141) of equal diameter distributed in multiple concentric rings around the disk axis, and liquid return notches (142) of the same structure and size located near the outer edge of the disk, with the center distances of the honeycomb holes (141) on each ring being equidistant in both circumferential and radial directions, and the outermost ring diameter of the honeycomb holes (141) being smaller than the inner diameter of the culture dish (13); the ratio of the radial size of the liquid return notches (142) to the maximum radius of disk A (14) is 0.02-0.05.

8. The flexible uniform flow culture dish automatic stirring device according to claim 5, **characterized in that**: the spherical disk (15) is provided with a central spherical surface (151) at the geometric center, multiple flow channel openings (152) at the outer edge, and multiple circumferential bosses (153) connected to the inner wall of the flow channel cylinder (17), with the distance ratio between the spherical disk (15) and disk A (14), and between the spherical disk (15) and disk B (16) being 0.65-0.95; the chord length of the central spherical surface (151) is the same as the inner diameter of the culture dish (13), and the spherical radius is determined by the inner diameter of the culture dish (13).

9. The flexible uniform flow culture dish automatic stirring device according to claim 5, **characterized in that**: the turbine (24) is a mixed flow structure with axial inflow and radial outflow, and has multiple wing-shaped blades (241), with the diameter of the flow channel inlet (242) of the turbine (24) being smaller than the flow channel hole at the center of disk B (16), with the rotating turbine (24) and the stationary disk B (16) coaxially aligned and closely fitted.

10. A method for using the flexible uniform flow culture dish automatic stirring device according to claim 5, comprising the following steps:
first, complete the sterilization of the flexible uniform flow culture dish automatic stirring device, close the one-way outlet valve (44), open the inlet valve (43) to inject distilled water into the outer tank (12) and the culture dish (13); turn on and adjust the speed of the power source (21), the turbine (24) rotates to stir the liquid in the culture dish (13) into the annular cavity between the outer tank (12) and the culture dish (13), and check if the monitoring system (3) is working properly;
second, after completing the preparation work, aseptically remove the outer tank cover plate (11) and evenly place fibrous carrier flocculent sheets in the culture dish (13), then seal and install the outer tank cover plate (11) on the upper end of the outer tank (12) again; reinject the nutrient solution into the culture dish (13) through the inlet valve (43), turn on and adjust the speed of the power source (21), the turbine (24) rotates to stir and push the nutrient solution in the culture dish (13) into the annular cavity between the outer tank (12) and the culture dish (13);
third, rotate and adjust the test tube (34) so that the probe of the pressure sensor (31) installed at the lower end of the test tube (34) is fully immersed in the nutrient solution, adjust the opening state of the outlet valve (44) and the speed of the turbine (24), to create a height difference between the nutrient solution inside the culture dish (13) and the nutrient solution in the annular cavity outside the culture dish (13);
fourth, adjust the test tube (34) so that the probe of the pressure sensor (31) is positioned at multiple horizontal levels in the culture dish (13), record the pressure data at different levels; when the monitored pressure value of the pressure sensor (31) exceeds the pressure tolerance level of the cultured cells, adjust the speed of the turbine (24) and the opening state of the outlet valve (44) according to the alarm displayed on the display screen (35); and
fifth, after a certain period of cell culture, use an external peristaltic pump to connect supply pipe A (41) and supply pipe B (42) to quantitatively replenish the nutrient solution; throughout the entire cell culture cycle, control the opening state of the outlet valve (44), the speed of the turbine (24), and the nutrient replenishment interval time to achieve multi-process cultivation of different cell types.
